# EUROPEAN PATENT APPLICATION

(11) **EP 1 362 598 A1**
(43) Date of publication of application: **19.11.2003**
(21) Application number: 02700593.3
(22) Date of filing: 18.02.2002
(51) Int. Cl.: A61K 41/00, A61K 45/00, A61K 33/44, A61K 31/409, A61K 47/48, A61K 47/32, A61K 47/34, A61K 47/36, A61P 43/00, A61P 35/00, A61P 31/12, A61P 33/10, A61P 11/00, A61P 1/16, A61P 13/12, A61P 9/10

(54) **ACTIVE OXYGEN GENERATOR CONTAINING PHOTOSENSITIZER FOR ULTRASONIC THERAPY**

(30) Priority: 19.02.2001 JP 2001041674
(71) Applicant: Tabata, Yasuhiko, Uji-shi, Kyoto 611-0024 (JP); MITSUBISHI CORPORATION, Tokyo 100-8086 (JP); THE HONJO CHEMICAL CORPORATION, Yodogawa-ku, Osaka 532-0003 Osaka-shi (JP)
(72) Inventor: TABATA, Yasuhiko, Uji-shi, Kyoto 611-0024 (JP)
(74) Representative: Becker Kurig Straus
(86) International application number: JP0201355
(87) International publication number: WO02066061

(57) **Abstract**

An active oxygen generator for ultrasonic therapy containing a photosensitizer of the present invention which can cause the photosensitizer such as fullerene to generate active oxygen even at deep sites within the body, thereby enabling it to be used for the treatment of deep cancers unable to be irradiated with light, as well as viral infections, intracellular parasitic infections, pulmonary fibrosis, liver cirrhosis, chronic nephritis, arteriosclerosis and angiostenosis.

## Description

### Technical Field

The present invention relates to an active oxygen generator containing a photosensitizer for ultrasonic therapy that causes a photosensitizer such as fullerene or porphyrin derivatives to generate active oxygen by irradiating with ultrasonic waves.

### Background Art

Since active oxygen such as singlet oxygen is highly reactive and exhibit various types of cytotoxicity such as severing of cellular DNA, inhibiting cell growth and inhibiting the activity of proteases, they are expected to demonstrate effects against various diseases such as cancer, viral infections, intracellular parasitic infections, pulmonary fibrosis, liver cirrhosis, chronic nephritis, arteriosclerosis and angiostenosis. This active oxygen is known to be generated by irradiating various types of photosensitizers with visible light, and examples of such photosensitizers include fullerene and porphyrin derivatives. However, since irradiation with light is essential for generating active oxygen in these photosensitizers, even in the case of the diseases listed above, its application is limited to those sites that can be irradiated with light (for example, cancers on the surface of the body or mucous membranes in the manner of skin cancer, tracheal cancer and esophageal epithelial cancer). For example, even if attempting to treat other diseases than those mentioned above, since the lesion is located inside organ tissue, irradiation with light has not always been effective. Consequently, there has been a need to develop an active oxygen generator containing a photosensitizer that is capable of causing active oxygen to be generated in a photosensitizer even in diseases at deep sites within the body that cannot be irradiated with light.

In addition, with respect to fullerene, which is a kind of photosensitizer, compounds are known that are generically referred to as Cₙ (carbon) clusters, examples of which include pure carbon substances such as C₆₀ or C₇₀ corresponding to the number n or carbon clusters that contain metals (or metal oxides) (see Chemistry, 50(6), 1995). Since fullerene itself is water-insoluble, it is difficult to administer into the body. On the other hand, substances with a high molecular size range are able to move easily and preferably to cancer tissue due to differences in its anatomical structure from that of normal tissue, and tend to remain for longer time period in cancer tissue. Consequently, studies have been conducted on the complexation of fullerene with various types of water-soluble polymers to increase the molecular size. This often results in reduction of adverse side effects which result from the cytotoxicity of active oxygen to normal tissue. Complexation with water-soluble polymers effectively enables fullerene to enhance the water-solubility as well as to increase the targetability to tumor tissues and prolong the remaining time period in cancer tissue. Examples of such water-soluble polymers for which the use in this manner has been proposed include polyethylene glycol, polyvinyl alcohol, dextran, pullulan, starch derivatives and their derivatives (see BIO INDUSTRY, Vol. 14, No. 7, pp. 30-37, 1997, and Japanese Provisional Patent Publication No. 235235/1997).

On the other hand, with respect to irradiation with ultrasonic waves, when a liquid is irradiated with ultrasonic waves, bubbles form within the liquid (cavitation), and when these formed bubbles are broken, heat, pressure and so forth are generated locally at that site. As a result, radicals (such as ·OH) are generated, and these radicals shift from an excited state to a basal state, or during re-bonding, light is known to be generated that has a wavelength range of primarily 300 to 600 nm (and this phenomenon is known as sonoluminescence) (see "Sonochemistry", K.S. Suslick, Science, Vol. 247, pp. 1439-1445, 1990).

As has been described above, there is a need to develop a system of an active oxygen generator containing a photosensitizer that is capable of generating active oxygen in fullerene and various other types of photosensitizers in the treatment of diseases located at deep sites in the body that cannot be irradiated with light. As a result of conducting extensive research in order to develop such a system, the inventors of the present invention found that the sonoluminescence is effective in enabling a photosensitizer to generate active oxygens since when the body is selectively irradiated with ultrasonic waves following administration of a photosensitizer to the body, the photosensitizer effectively generates active oxygen due to light having a wavelength range of primarily 300 to 600 nm, thereby leading to completion of the present invention.

### Disclosure of the Invention

The present invention relates to an active oxygen generator for ultrasonic therapy that contains a photosensitizer such as fullerene complexed with a water-soluble polymer.

As was previously described, the active oxygen generator for ultrasonic therapy of the present invention causes a photosensitizer to generate active oxygen within the body by utilizing sonoluminescence (light generation phenomenon) that occurs when a photosensitizer is irradiated with ultrasonic waves, which in turn enables it to destroy the cells of lesions such as cancer, viral infections, intracellular parasitic infections, pulmonary fibrosis, liver cirrhosis, chronic nephritis, arteriosclerosis and angiostenosis.

### Brief Description of the Drawings

Fig. 1 indicates an example of the structure of fullerene bonded to a water-soluble polymer that is contained as a photosensitizer in the active oxygen generator for ultrasonic therapy of the present invention.
Fig. 2 indicates the cell survival rates of RL♂1 cells in the case of not adding fullerene-PEG-NH₂ conjugate to the wells of a microplate.
Fig. 3 indicates the cell survival rates of RL♂1 cells in the case of adding fullerene-PEG-NH₂ conjugate at 5 µg/well.
Fig. 4 indicates the cell survival rates of RL♂1 cells in the case of adding fullerene-PEG-NH₂ conjugate at 5 µg/well.
Fig. 5 indicates the cell survival rates of RL♂1 cells in the case of adding fullerene-PEG-NH₂ conjugate at 0 to 10 µg/well.
Fig. 6 indicates the administration of fullerene-PEG-NH₂ conjugate to tumor-bearing mice and the effects of ultrasonic therapy following administration.

### Best Mode for Carrying out the Invention

Any photosensitizer can be used for the photosensitizer contained in the active oxygen generator for ultrasonic therapy of the present invention provided it is capable of generating active oxygen by sonoluminescence, specific examples of which include fullerene and various types of porphyrin derivatives complexed with a water-soluble polymer, as well as conventionally used photosensitizers such as acridine, rose Bengal, acridine orange, berberine sulfate, fluorescein, tetracycline, eosin Y, NTS, psoralen, boneline, pheoformide, chlorin e 6, mesotetra (hydroxyphenyl)chlorin, phthalocyanine, purpurin, 5-aminolaebrinic acid (ALA), HAT-DO1 (magnesium chlorine-chlorine dimer) and various other photosensitizers. Examples of porphyrin derivatives include porfimer sodium (trade name: Photofrin), hematoporphyrin, metalloporphyrin, tetraphenylporphyrin sulfate, protoporphyrin, uroporphyrin, coproporphyrin, dihematoporphyrin ether (DHE), benzoporphyrin (BPS), ATX-70 (gallium-porphyrin complex) and ATX-S10 (four-formyloximethylidene-3-hydroxy-2-vinyldeuterio porphynyl(IX)-6-7-bisaspartic acid). Among the various types of porphyrin derivatives, porfimer sodium can be used particularly preferably.

The fullerene complexed with a water-soluble polymer, which is one of the photosensitizers contained in the active oxygen generator for ultrasonic therapy of the present invention, is the product of complexing inherently water-insoluble fullerene with a water-soluble polymer by chemical bonding or physical bonding using intermolecular force, and water solubility is imparted by the complexation with the water-soluble polymer. There are no particular restrictions on the type of fullerene, and although any type can be used provided it generates active oxygen, the n=60 pure carbon substance of C₆₀ fullerene, C₇₀ fullerene, the pure carbon substance of nanotube fullerene and various types of higher fullerenes can be used. From the viewpoints of ease of supply and handling, nanotubes and C₆₀ fullerene are used preferably. Nanotubes (Fullerene Chemistry and Physics, Shinohara et al., Nagoya University Press) can be used particularly preferably since they are finer than conventional carbon fibers, consist nearly entirely of graphite (each of the graphite layers are laminated in the form of a nested structure), the ends are closed due to the entry of five-member rings, and each layer has a spiral structure. Each of these types of fullerene are available commercially, and can be acquired from manufacturers such as Honjo Chemical Corp., Mitsubishi Corp. and Tokyo Chemical Industry Co., Ltd. (under the trade names of, for example, C₆₀ Fullerene, C₇₀ Fullerene, Multi-Wall Nanotube and Single-Wall Nanotube).

In addition, there are no particular restrictions on the water-soluble polymer complexed with the fullerene to impart water-solubility thereto, and various types of commercially available water-soluble polymers may be used. Specific examples of water-soluble polymers that can be used include non-ionic water-soluble synthetic polymers such as polyethylene glycol, polypropylene glycol, polyvinyl alcohol and polyvinyl pyrrolidone; dextran; pullulan; non-ionic water-soluble polymers including starch derivatives such as starch, hydroxyethyl starch and hydroxypropyl starch; arginic acid; hyaluronic acid; chitosan; chitin derivatives; anionic or cationic derivatives of these polymers, and copolymers having two or three of these polymers as components. Polyethylene glycol can be used preferably since it has common solvents with fullerene, functional groups involved in the compounding reaction with fullerene are located only on the ends of the molecule, and the chemical bonding mode is simple.

Although there are no particular restrictions on the molecular weight of these water-soluble polymers, those can be used having a molecular weight of 1,000 to 1,000,000, and preferably 5,000 to 50,000.

Polyethylene glycol having a molecular weight of 1,000 to 1,000,000, and particularly 5,000 to 15,000, is used particularly preferably for the water-soluble polymer.

A water-soluble polymer that can be used to complex with the fullerene in the active oxygen generator for ultrasonic therapy of the present invention has functional groups for compounding with fullerene, and the water-soluble polymer compounds with fullerene by means of those functional groups. Any functional groups that enable compounding with fullerene can be used for the functional groups, examples of which include functional groups having nucleophilic substitution reactivity such as an amino group, hydroxy group, cyano group and carboxyl group. Amino groups can be used preferably. For example, in the case of bonding fullerene with a water-soluble polymer by chemical bonding using amino groups, the fullerene is bonded with the water-soluble polymer by an addition reaction of the amino groups to the double bond of fullerene. Although such functional groups may be present at any location within the water-soluble polymer molecule provided it is a location suitable for compounding with fullerene, in consideration of the ease of compounding, they are preferably located on the ends of the water-soluble polymer. In the case of using a water-soluble polymer that does not have such functional groups, it is necessary to first introduce such functional groups prior to complexation with fullerene. Moreover, in addition to functional groups such as amino groups, the water-soluble polymer that can be used to compound with fullerene also preferably has functional groups that do not react with fullerene, such as a C₁₋₆ alkoxy group e.g. a methoxy. In the case where the water-soluble polymer has a functional group like an amino group on one end of the polymer, and where a functional group that bonds with fullerene is also present on the other end, an aggregate of fullerene and water-soluble polymers ends up being formed due to compounding by both ends of the water-soluble polymer with fullerene, such aggregates will not often have sufficient water solubility since the water-solubilization of molecular aggregate is not practically sufficient. Thus, the water-soluble polymer that can be used to complex with fullerene preferably additionally has a functional group like a methoxy group that does not react with fullerene in addition to a functional group such as an amino group. However, even if the water-soluble polymer is polyethylene glycol which has functional groups like an amino group on both ends, as long as the resulting aggregate of fullerene and water-soluble polymer has adequate water solubility, such a water-soluble polymer can still be used.

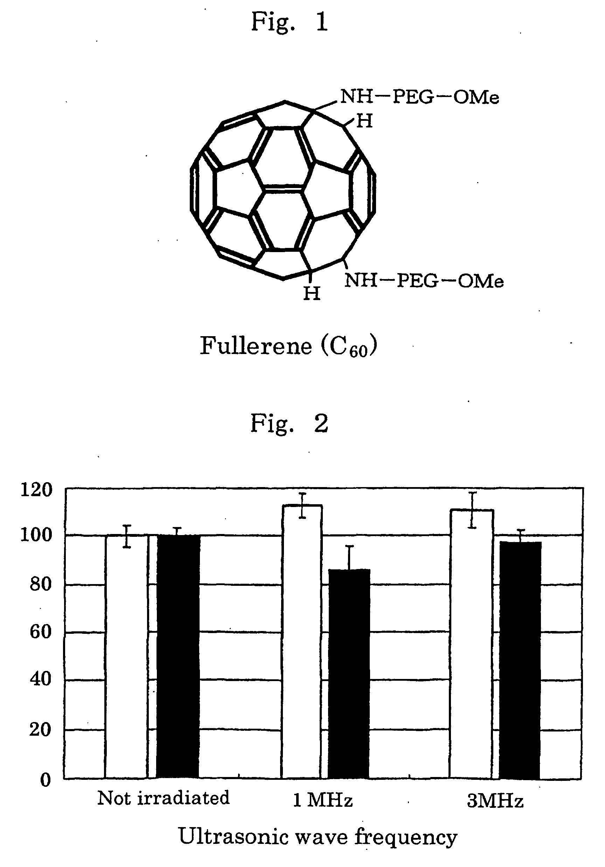

An example of the structure of fullerene bonded with a water-soluble polymer that is a photosensitizer contained in the active oxygen generator for ultrasonic therapy of the present invention is shown in Fig. 1. The examples shows the case of bonding polyethylene glycol having an amino group on one end and a methoxy group on the other end with C₆₀ fullerene.

As was previously described, the fullerene complexed with the water-soluble polymer contained as photosensitizer in the active oxygen generator for ultrasonic therapy of the present invention should have water solubility to a degree that permits administration into the body. In the case of low water solubility, although fullerene that has been complexed with water-soluble polymer forms an aggregate mass, since the particle diameter of the mass is required to be no larger than 400 nm in consideration of the ease of migration and accumulation in cancer and other tissue, water solubility is required to a degree that prevents the formation of an aggregate mass larger than this size. The blending molar ratio of fullerene to water-soluble polymer required to achieve this degree of solubility varies according to the type of water-soluble polymer used, and the content of amino groups and other functional groups in the water-soluble polymer molecules. For example, in the case of using polyethylene glycol having one amino group per molecule and a molecular weight of 5,000 to 15,000, the blending molar ratio is preferably within the range of 1:0.1 to 1:150. A molar ratio of 1:50 to 1:150 is particularly preferable for obtaining satisfactory water solubility.

In the production of the active oxygen generator for ultrasonic therapy of the present invention that contains fullerene chemically bonded with a water-soluble polymer as a photosensitizer, fullerene and functional group-containing water-soluble polymer blended at a molar ratio required for achieving the desired solubility are dissolved in an organic solvent and stirred under protection from light to bond the fullerene and water-soluble polymer by means of the functional groups. Examples of organic solvents include benzene, dimethylsulfoxide, tetrahydrofuran and N,N-dimethylformamide, and benzene can be used preferably. The reaction temperature is about 4 to 40°C, and preferably about 25°C, while the reaction time is about 6 to 48 hours, and preferably about 24 hours. The resulting reaction product can be recovered by freeze-drying after purifying by hydrophobic or affinity chromatography. In addition, in the production of the active oxygen generator for ultrasonic therapy containing fullerene physically bonded with a water-soluble polymer by intermolecular force, the fullerene and functional group-containing water-soluble polymer should be mixed at a molar ratio required to achieve the desired solubility. In this case as well, the aggregate mass obtained by mixing preferably has a particle diameter of no larger than 400 nm from the viewpoint of ease of migration into tissue.

Furthermore, as was previously mentioned, the water-soluble polymer used to produce the active oxygen generator for ultrasonic therapy of the present invention that contains fullerene complexed with water-soluble polymer as a photosensitizer is required to have a functional group such as an amino group to enable it to compound with fullerene. In the case of using a water-soluble polymer that does not have a functional group, it is necessary to first introduce a functional group before compounding with fullerene. For example, in order to introduce an amino group into a water-soluble polymer having only a hydroxy group, a chemical bond is formed between the hydroxy group of the water-soluble polymer and an amino compound having two or more amino groups in a single molecule such as an alkyldiamine, lysine or lysine ester compound by the periodic acid oxidation method, cyanuryl chloride method, cyan bromide method or epichlorhydrin method, and the amino group is introduced into the polymer side chain. In addition, in the case of a water-soluble polymer having a carboxyl group, an amino group is introduced into the polymer side chain by a bonding reaction between the carboxyl group and an amino compound using, for example, N-hydroxysuccinimide-carbodiimide, carbodiimide or ethylchlorocarbonate. For example, in the case of introducing an amino group into polyethylene glycol, the polyethylene glycol having a COOH group on both ends is dissolved in pH 5.0 phosphate buffer (10% by weight) followed by the addition of 3 moles of water-soluble carbodiimide relative to the COOH groups, and by then stirring for 1 hour at room temperature, the carboxyl groups are activated. Subsequently, 10 moles of ethylene diamine are added relative to the COOH groups and allowed to additionally react for 6 hours at room temperature. Polyethylene glycol in which amino groups have been introduced on both ends can then be obtained by dialyzing the resulting reaction solution against water. In addition, polyethylene glycol in which an amino group has been introduced on one end and a methoxy group has been introduced on the other end can be acquired from NOF Corporation.

The active oxygen generator for ultrasonic therapy of the present invention containing fullerene complexed with a water-soluble polymer as photosensitizer obtained in the manner described above has adequate water solubility for administering to the body while also exhibiting a high degree of migration and retention in cancer tissue and inflammatory tissue as a result of compounding the fullerene with the water-soluble polymer.

Although the photosensitizer contained in the active oxygen generator for ultrasonic therapy of the present invention has a degree of water solubility that enables it to be administered into the body, photosensitizers other than fullerene complexed with water-soluble polymer can also be enhanced for improved accumulation and retention in cancer tissue and inflammatory tissue by bonding with water-soluble polymer as necessary. In this case, the same various types of water-soluble polymers as listed for the water-soluble polymer complexed with fullerene can be used. These photosensitizers can be chemically bound with water-soluble polymer using a method conventionally used in the field of organic chemistry.

Since photosensitizers such as fullerene contained in the active oxygen generator for ultrasonic therapy of the present invention exhibit cytotoxicity due to the generation of singlet oxygen and other forms of active oxygen in an aqueous medium by light generated as a result of sonoluminescence induced by irradiation with ultrasonic waves, they can be used to treat various diseases including cancer. The radiated ultrasonic waves are preferably used at a frequency of about 100 KHz to 20 MHz, and particularly preferably at about 1 to 3 MHz. Irradiation is preferably performed at an output of about 0.1-5 watts/cm², and particularly preferably about 2 watts/cm². The duty cycle is about 1 to 100%, and preferably about 10%. Although varying according to the frequency and irradiation output used, the irradiation time is about 5 to 300 seconds, and preferably abut 30 to 120 seconds.

The active oxygen generator for ultrasonic therapy of the present invention is effective for treatment of all types of cancer, viral infections, intracellular parasitic infections, pulmonary fibrosis, liver cirrhosis, chronic nephritis, arteriosclerosis and angiostenosis for which active oxygen exhibits cytotoxicity. Examples of cancers include all solid cancers that occur in the surface layer and interior of organs in the manner of lung cancer, liver cancer, pancreatic cancer, gastric cancer, urinary bladder cancer, kidney cancer and brain tumors. In particular, the active oxygen generator for ultrasonic therapy of the present invention can also be effectively used for treatment of deep cancers which cannot be irradiated with light and for which conventional photodynamic therapy is inapplicable. With respect to other diseases, since the lesion or infected cells (affected cells) are located within an organ, after accumulating the photosensitizer at that site by a suitable method, treatment can be performed by irradiating that site with ultrasonic waves from the outside.

The active oxygen generator for ultrasonic therapy of the present invention can be in any dosage form such as an injection (intravenous, intra-arterial, intramuscular, subcutaneous or intracutaneous), dispersion, liquid or solid powder. For example, in the case of using in the form of an injection, an injection preparation can be prepared by blending a buffer, physiological saline, preservative, distilled water for injection or various other additives typically used for injections with the active oxygen generator for ultrasonic therapy of the present invention. Although varying according to the administration route, age and sex of the patient and type and state of the disease, the adult dosage of the active oxygen generator for ultrasonic therapy of the present invention is about 1 to 10 mg/kg per day, and this dosage can be administered in a single administration or by dividing into several administrations.

As was previously described, substances with a high molecular size range has a targetability to tumor tissues and inflammatory tissue and a prolonged remaining time period more than normal tissue. Thus, when the active oxygen generator for ultrasonic therapy of the present invention that contains a photosensitizer complexed or bonded with a water-soluble polymer is administered to the body, it is accumulated in cancer tissue and inflammatory tissue more than in normal tissue, and is retained in cancer tissue and inflammatory tissue at a higher concentration and for a longer duration than in normal tissue. On the other hand, since the active oxygen generator for ultrasonic therapy of the present invention is excreted more rapidly in normal tissue than in cancer tissue and inflammatory tissue, if a certain amount of time is allowed to elapse after administration of the active oxygen generator for ultrasonic therapy of the present invention is administered to the body, the concentration of the active oxygen generator for ultrasonic therapy of the present invention in cancer tissue and inflammatory tissue becomes significantly higher than the concentration in normal tissue, and the active oxygen generator for ultrasonic therapy of the present invention becomes specifically distributed at high concentrations in cancer tissue and inflammatory tissue. Thus, if the body is irradiated with ultrasonic waves a certain amount of time after administration of the active oxygen generator for ultrasonic therapy of the present invention to the body, the photosensitizer is made to generate singlet oxygen and other forms of active oxygen due to the light generated by sonoluminescence induced by irradiation with ultrasonic waves, thereby resulting in the demonstration of antitumor activity and antiinflammatory activity specifically in cancer tissue and inflammatory tissue. In normal tissue, on the other hand, since the concentration of the active oxygen generator for ultrasonic therapy of the present invention is lower, the cytotoxicity with respect to normal tissue is not as high as that targeted at cancer tissue and inflammatory tissue, and adverse side effects in normal tissue are therefore expected to be alleviated.

Following administration of the active oxygen generator for ultrasonic therapy of the present invention in humans, the concentration of photosensitizer in cancer tissue and inflammatory tissue becomes significantly higher than that in normal tissue, and although varying according to metabolic state at the treatment site of the individual patient, time-based changes in the distribution of photosensitizer and so forth, the time until ultrasonic irradiation becomes possible is typically 0.1 to 48 hours after administration, and in particular, ultrasonic irradiation is preferably performed about 24 hours after administration. When irradiating a human body with ultrasonic waves, ultrasonic waves at the frequency previously mentioned are radiated at the output and duration previously described. Thus, in performing treatment using the active oxygen generator for ultrasonic therapy of the present invention, the active oxygen generator for ultrasonic therapy of the present invention is administered to the patient in the form of, for example, an injection, and ultrasonic irradiation is performed using an ultrasonic wave generator about 0.1 to 48 hours after administration. The dosage, frequencies of administration and irradiation, number of treatments and so forth can be determined according to such factors as the age, body weight and sex of the patient, and the type and state of the disease.

In addition, although the active oxygen generator for ultrasonic therapy of the present invention that contains a photosensitizer not complexed or bonded with a water-soluble polymer does not specifically distribute and accumulate in cancer tissue and inflammatory tissue, by using an arbitrary method for delivering the active oxygen generator for ultrasonic therapy of the present invention to target tissue or cells such as a method for specifically delivering to target tissue or cells using a drug delivery system, cytotoxic action can be demonstrated in the target tissue or cells. Examples of such methods include a method in which the active oxygen generator for ultrasonic therapy of the present invention is injected directly to the target tissue or cells (enabling delivery to nearly all sites in the body by using, for example, an endoscope), and a method in which the active oxygen generator for ultrasonic therapy of the present invention is administered after complexing an antibody with the target tissue or cells, lectin, cell adhesion factor, sugar chain or other cell recognition factors to a photosensitizer. In addition, cytotoxicity can be demonstrated by generating active oxygen only at a desired site by administering the active oxygen generator for ultrasonic therapy of the present invention to the body followed by irradiating only the site where the photosensitizer is desired to generate active oxygen with ultrasonic waves. In addition, the selectivity of the site where cytotoxicity is to be demonstrated can be improved by focusing the ultrasonic waves.

### Examples

Although the following provides a detailed explanation of the production method of the active oxygen generator for ultrasonic therapy of the present invention along with its antitumor activity, the active oxygen generator for ultrasonic therapy of the present invention is not limited as a result of this, and this explanation is applied similarly to photosensitizers other than the described photosensitizers as well as to diseases other than the described diseases.

### Production Example 1: Production of Active Oxygen Generator for Ultrasonic Therapy of the Present Invention

Polyethylene glycol having an amino group on one end and a methoxy group on the other end (abbreviated as PEG-NH₂, molecular weight: about 5,000, NOF Corp.) and C₆₀ fullerene (Tokyo Chemical Industry Co., Ltd.) were used for the water-soluble polymer. 10 ml of a benzene solution containing 0 to 108 mM PEG-NH₂ were added to 10 ml of a benzene solution containing 0.54 mM C₆₀ fullerene, and the fullerene was bonded with the PEG-NH₂ by stirring for 24 hours at 25°C under protection from light to obtain fullerene-PEG-NH₂ conjugate. Following completion of the reaction, the reaction solution was freeze-dried, and the fullerene-PEG-NH₂ conjugate was extracted with water to investigate its migration into water by dissolving the fullerene-PEG-NH₂ conjugate in benzene to a fullerene concentration of 0.27 mM, mixing with an equal volume of distilled water and then allowing to stand for 48 hours at 25°C. Migration into water was evaluated by measuring the change in optical absorbance of fullerene at 500 nm of benzene solutions before and after extraction. As a result, the migration of the fullerene-PEG-NH₂ conjugate into water was observed to increase the greater the ratio of PEG-NH₂ to fullerene. On the basis of this finding, it was indicated that, although fullerene is inherently insoluble in water, by bonding with PEG-NH₂, its water solubility increases thereby enabling the fullerene to become water-soluble. In the case the molar ratio of the added PEG-NH₂ relative to the fullerene was 50 or more, nearly 100% of the fullerene migrated into the aqueous phase, and nearly complete solubilization of the fullerene was achieved.

### Test Example 1: In Vitro Antitumor Activity

Cancer cells, RL♂1 cells (provided by the Kyoto Pasteur Research Laboratory) were cultured to a confluent in RPMI-1640 medium (Cosmo Bio, 305-02-01, containing 10% serum) using 100 mm dishes under culturing conditions consisting of 5% CO₂, 95% air and 37°C.

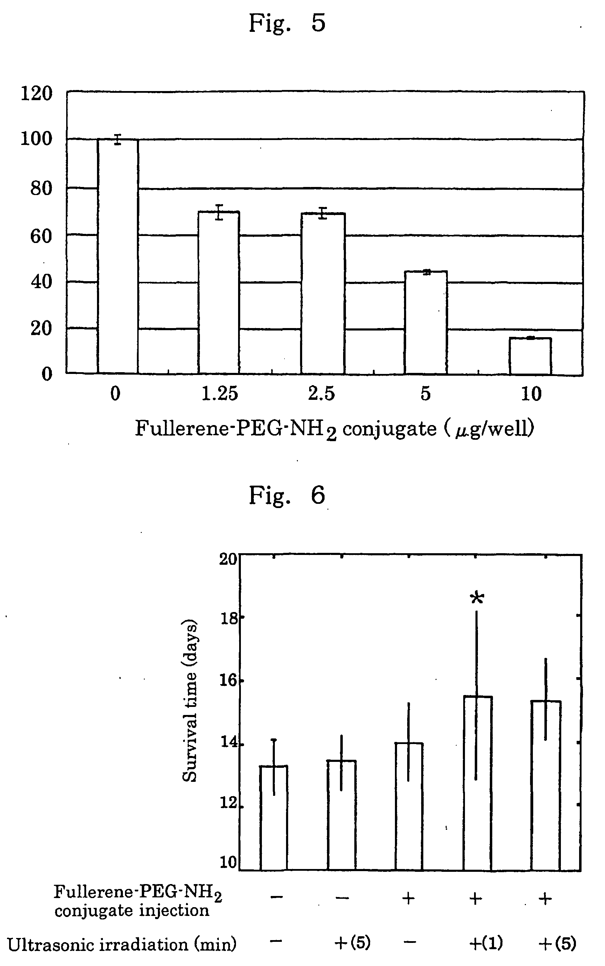

Fullerene-PEG-NH₂ conjugate produced in the same manner as Production Example 1 was dissolved in a media having the same composition as that used to culture the cancer cells under protection from light, and after adjusting to a concentration of 10 µg/ml, was sterilized and filtered. One ml aliquots of the media containing the adjusted fullerene-PEG-NH₂ conjugate were added to each well of a six-well plate. Only media not containing the conjugate was added to a control well. The above cell confluent was adjusted to contain 2 x 10⁵ cells/ml to obtain a cell suspension, and 1 ml aliquots of this suspension were added to each of the above wells. (At this point, each well contained 10 µg of fullerene-PEG-NH₂ conjugate, 2 x 10⁵ RL♂1 cells and 2 ml of media.) After gently agitating the cells in the wells with a pipette, the wells were irradiated with ultrasonic waves using an ultrasonic wave irradiation system (Williams Healthcare Systems, Model #6100) from the bottoms of the 6-well plates by means of conductive gel (frequency: 1 MHz, output: 2 watts/cm², irradiation time: 60 seconds, duty cycle: 10%). Following irradiation with ultrasonic waves, the plates were protected from light with aluminum foil and cultured for 3 days in an incubator (37°C, 5% CO₂). Subsequently, the number of viable cells was measured using a cell counting device (Cell Counting Kit, Dojin Chemical, 345-06463) followed by calculation of the proportion of the number of viable cells to a control, to which fullerene-PEG-NH₂ conjugate was not added and which was not irradiated with ultrasonic waves, as a percentage. As a result, as shown in Fig. 5, the number of viable cells was 20% or less as compared with the control in the presence of fullerene-PEG-NH₂ conjugate at 10 µg/well. Moreover, the number of viable cells was also counted while changing the amount of fullerene-PEG-NH₂ conjugate added to the wells to 1.25, 2.5, 5 or 10 µg/ml, changing the frequency of the ultrasonic waves to 1 or 3 MHz, and changing the irradiation time between 0 and 120 seconds. The output and duty cycle were left unchanged. Those results are shown in Figs. 2 through 5.

Fig. 2 shows the cell viable rate for RL♂1 cells in the case of not adding fullerene-PEG-NH₂ conjugate to the wells (frequency: 1 or 3 MHz, irradiation time: 30 seconds with a white bar and 60 seconds with a black bar). The number of viable cells did not change for irradiation at 1 MHz for 60 seconds.

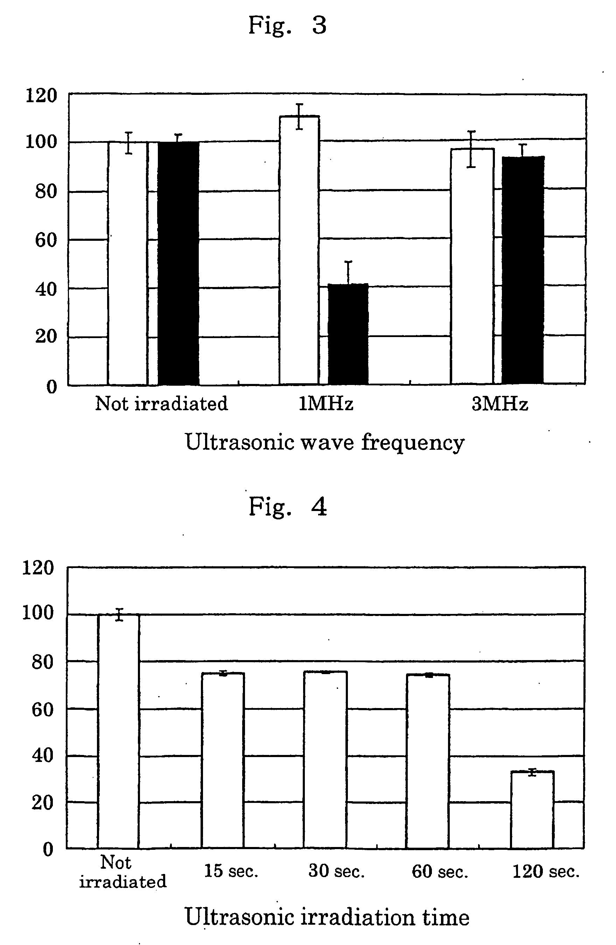

Fig. 3 shows the cell survival rate for RL♂1 cells in the case of adding fullerene-PEG-NH₂ conjugate at 5 µg/well (frequency: 1 or 3 MHz, irradiation time: 30 seconds with a white bar, 60 seconds with a black bar). The number of viable cells was observed to decrease significantly for irradiation at 1 MHz for 60 seconds (p<0.05 relative to 30 seconds).

Fig. 4 shows the cell survival rate in the case of adding fullerene-PEG-NH₂ conjugate at 5 µg/well and varying the 1 MHz ultrasonic wave irradiation time between 0 and 120 seconds. The cell survival rate was observed to decrease as the irradiation time was lengthened. In addition, the cell survival rate decreased significantly starting at an irradiation time of 15 minutes.

Fig. 5 shows the cell survival rate in the case of varying the amount of fullerene-PEG-NH₂ conjugate added between 0 to 10 µg/well (frequency: 1 MHz, irradiation time: 60 seconds). The cell survival rate was observed to decrease as the amount of fullerene-PEG-NH₂ conjugate added increased. A significant difference was observed starting at a fullerene-PEG-NH₂ conjugate concentration of 1.25 µg/well.

### Test Example 2: In Vivo Antitumor Activity

In order to prepare tumor-bearing mice, 5 x 10⁶ mouse lymphoma (RL♂1) cells were suspended in 100 µl of RPMI-1640 medium and administered into the caudal vein of BALB/C mice. These mouse lymphoma cells are used as a model in which tumors are formed in the liver of mice in about 10 days after administration and cause the mice to die of cancer. The fullerene-PEG-NH₂ conjugate was produced in the same manner as described in Production Example 1, and an aqueous solution of a fullerene-PEG-NH₂ conjugate containing 400 µg as fullerene was administered into the caudal vein of the mice 24 hours (1 day) after administration of the lymphoma cells. An irradiation probe was placed against the skin 24 hours after administration, and the site corresponding to the liver was irradiated with ultrasonic waves for 60 seconds from outside the body (frequency: 1 MHz, output: 2 watts/cm², duty cycle: 10%), after which the day on which each mouse died was recorded to determine the survival rate.

As a result, an increase in the mouse survival rate was observed due to irradiation with ultrasonic waves. In the mouse group that was not irradiated with ultrasonic waves following administration of the fullerene-PEG-NH₂ conjugate, the mouse group that was only irradiated with ultrasonic waves but not administered the fullerene-PEG-NH₂ conjugate, and the mouse group in which neither administration or irradiation was performed, all of the mice died about 14 days after administration of lymphoma cells. In the mouse groups that were irradiated with ultrasonic waves following administration of fullerene-PEG-NH₂ conjugate, the mice in all of the groups survived regardless of irradiation time even at 14 days after administration of lymphoma cells, and the effect was considered to be enhanced as the duration of ultrasonic wave irradiation time became longer.

### Test Example 3: Active Oxygen Generation Test

The amount of active oxygen (superoxide anion, O₂⁻) generated was measured using the cytochrome method. 800 µl of Hanks solution (HBSS, pH = 7.4, Life Technologies Oriental, Inc., Tokyo) containing 30 µM cytochrome C were mixed with 200 µl of an HBSS solution of the C₆₀ fullerene-PEG-NH₂ conjugate obtained in Production Example 1 to a final conjugate concentration of 2.5 µg/ml. This solution was then irradiated with ultrasonic waves for 60 seconds (output: 2 watts/cm², frequency: 1 MHz, duty cycle: 10%). After allowing to stand for 5 minutes at 25°C, the optical absorbance of the solution at 550 nm was measured. Each procedure was carried out in a dark location, and the experiment was conducted three times for each group. The amounts of O₂⁻ generated after irradiating with ultrasonic waves in the presence and absence of C₆₀ fullerene-PEG-NH₂ conjugate are shown in the table below. Significant O₂⁻ generation was observed only in the case of performing ultrasonic irradiation in the presence of C₆₀ fullerene-PEG-NH₂ conjugate. There were hardly any changes in O₂⁻ generation both in the case of not performing ultrasonic irradiation in the presence of C₆₀ fullerene-PEG-NH₂ conjugate and the case of performing ultrasonic irradiation in the absence of C₆₀ fullerene-PEG-NH₂ conjugate.

| Generation of O₂⁻ by C₆₀ Fullerene-PEG-NH₂ Conjugate | | |
|---|---|---|
| C₆₀ fullerene-PEG-NH₂ conjugate (µg/ml) | Ultrasonic irradiation^{a)} | O₂⁻ generation (µM) |
| 0 | Irradiated | ND^{b)} |
| 0 | Not irradiated | ND |
| 2.5 | Irradiated | 2.38±0.08^{*c)} |
| 2.5 | Not irradiated | 0.32±0.02 |

| | | |
|---|---|---|
| a) Irradiation time: 60 seconds, output: 2 watts/cm², frequency: 1 MHz, duty cycle: 10% | | |
| b) ND: Not detected | | |
| c) Mean ± SE *: p<0.05, Significant difference relative to amount of O₂⁻ generated in the presence of C₆₀ fullerene-PEG-NH₂ conjugate in the case of not irradiating with ultrasonic waves. | | |

### Test Example 4: Sonic Dynamics Effect of C₆₀ Fullerene-PEG-NH₂ Conjugate on Tumors

In order to acclimate RL♂1 cells to in vivo conditions, female six-week-old BALB/c mice (Japan SLC, Shizuoka) were inoculated intravenously with the cells at a concentration of 5 x 10⁶ cells/0.2 ml media/mouse. Two weeks later, the livers of the mice were extracted and lymphoma cells were isolated from the nodes that formed in the liver. The mice were again inoculated in the same manner with the isolated cells. Next, mice were intravenously inoculated with 0.2 ml of the resulting RL♂ 1 cells (5 x 10⁶ cells/mouse) to obtain mice with liver tumors.

One day after tumor inoculation, PBS containing the C₆₀ fullerene-PEG-NH₂ conjugate obtained in Production Example 1 were intravenously administered to the tumor-bearing mice at 400 µg/0.2 ml/mouse. Thirty minutes later, the liver was irradiated with ultrasonic waves for 1 or 5 minutes (output: 2 watts/cm², frequency: 1 MHz, duty cycle: 20%). An ultrasonic transducer chip was attached to the skin of the abdomen, and the liver was irradiated with ultrasonic waves transcutaneously by means of ultrasonic conductive gel. The mice were observed daily and their survival times were recorded. For a control group, the mice were intravenously injected with polyethylene glycol not containing C₆₀ fullerene followed by irradiation or non-irradiation with ultrasonic waves. In addition, an experiment in which only the C₆₀ fullerene-PEG-NH₂ conjugate was administered without ultrasonic irradiation, and an experiment in which only ultrasonic irradiation was performed without injecting C₆₀ fullerene-PEG-NH₂ conjugate, were also performed. Six mice were used in each experiment, and although all of the mice were given free access to ordinary laboratory rodent diet and drinking water, they were housed in a dark room for the duration of the experiments.

The results are shown in Fig. 6. Fig. 6 illustrates the administration of C₆₀ fullerene-PEG-NH₂ conjugate to tumor-bearing mice along with the therapeutic effect resulting from subsequent ultrasonic irradiation. The mouse survival times were not extended in the case of only administering C₆₀ fullerene-PEG-NH₂ conjugate and in the case of only performing ultrasonic irradiation. Conversely, in the case of administering the C₆₀ fullerene-PEG-NH₂ conjugate followed by ultrasonic irradiation, the mouse survival times were significantly extended in the case of irradiating the liver with ultrasonic waves for 1 minute.

### Industrial Applicability

Since the active oxygen generator for ultrasonic therapy of the present invention generates singlet oxygen and other forms of active oxygen by irradiating the body with ultrasonic waves following administration, it can be used effectively for the treatment of deep cancers that can not be irradiated with light, such as lung cancer, liver cancer, pancreatic cancer, gastric cancer, urinary bladder cancer, kidney cancer and brain cancer, as well as viral infections, intracellular parasitic infections, pulmonary fibrosis, liver cirrhosis, chronic nephritis, arteriosclerosis and angiostenosis.

## Claims

1. An active oxygen generator for ultrasonic therapy containing a photosensitizer.

2. The active oxygen generator for ultrasonic therapy according to claim 1, wherein the photosensitizer is fullerene complexed with a water-soluble polymer.

3. The active oxygen generator for ultrasonic therapy according to claim 2, wherein the fullerene is C₆₀ fullerene or nanotube fullerene.

4. The active oxygen generator for ultrasonic therapy according to claim 2 or claim 3, wherein the water-soluble polymer is selected from a non-ionic water-soluble synthetic polymer such as polyethylene glycol, polypropylene glycol, polyvinyl alcohol or polyvinyl pyrrolidone; dextran; pullulan; a non-ionic water-soluble polymer including starch derivatives such as starch, hydroxyethyl starch or hydroxypropyl starch; arginic acid; hyaluronic acid; chitosan; chitin derivatives; anionic or cationic derivatives of these polymers, and copolymers having two or three of these polymers as components.

5. The active oxygen generator for ultrasonic therapy according to anyone of claims 2 through 4, wherein the water-soluble polymer is complexed with fullerene by means of a functional group.

6. The active oxygen generator for ultrasonic therapy according to claim 5, wherein the functional group is an amino group.

7. The active oxygen generator for ultrasonic therapy according to claim 1, wherein the photosensitizer is a porphyrin derivative.

8. The active oxygen generator for ultrasonic therapy according to claim 7, wherein the porphyrin derivative is bonded with a water-soluble polymer.

9. The active oxygen generator for ultrasonic therapy according to claim 8, wherein the water-soluble polymer is selected from a non-ionic water-soluble synthetic polymer such as polyethylene glycol, polypropylene glycol, polyvinyl alcohol or polyvinyl pyrrolidone; dextran; pullulan; a non-ionic water-soluble polymer including starch derivatives such as starch, hydroxyethyl starch or hydroxypropyl starch; arginic acid; hyaluronic acid; chitosan; chitin derivatives; anionic or cationic derivatives of these polymers, and copolymers having two or three of these polymers as components.

10. A therapeutic agent for cancer, viral infections, intracellular parasitic infections, pulmonary fibrosis, liver cirrhosis, chronic nephritis, arteriosclerosis and angiostenosis containing the active oxygen generator for ultrasonic therapy according to anyone of claims 1 through 9.

11. A pharmaceutical composition containing the active oxygen generator for ultrasonic therapy according to anyone of claims 1 through 9, and a pharmaceutically acceptable carrier.
